# EUROPEAN PATENT APPLICATION

(11) **EP 3 973 937 A1**
(43) Date of publication of application: **30.03.2022**
(21) Application number: 20833388.0
(22) Date of filing: 22.06.2020
(51) Int. Cl.: A61F 13/53, A61F 13/534, A61F 13/535, A61F 13/537

(54) **ABSORBENT ARTICLE**

(30) Priority: 26.06.2019 JP 2019118641
(71) Applicant: Zuiko Corporation, Settsu-shi Osaka 566-0045 (JP)
(72) Inventor: FURUKAWA, Daisuke, Settsu-shi, Osaka 566-0045 (JP); IKEDA, Natsuki, Settsu-shi, Osaka 566-0045 (JP)
(74) Representative: Müller-Boré & Partner Patentanwälte PartG mbB
(86) International application number: PCT/JP2020/024295
(87) International publication number: WO 2020/262274

(57) **Abstract**

Provided is an absorbent article capable of fully utilizing absorption capacity of an absorption layer downstream in a liquid supply direction in a structure in which a plurality of absorption layers are laminated. An absorbent article (1) includes a first absorption layer (2a) and a second absorption layer (3a) configured to absorb liquid, and a diffusion layer (2b) that is interposed between the first absorption layer (2a) and the second absorption layer (3a) and diffuses the liquid in a direction in which a plane of the second absorption layer (3a) spreads. The first absorption layer (2a) includes an absorbent resin configured to absorb liquid. The first absorption layer (2a) includes an absorption region (2a1) including the absorbent resin, and a passage region (6) that has a lower density of the absorbent resin than a density of the absorbent resin in the absorption region (2a1). The passage region (6) allows the liquid to pass through the diffusion layer (2b).

## Description

### Technical Field

The present invention relates to an absorbent article that absorbs liquid.

### Background Art

Conventionally, there are various absorbent articles as absorbent articles that absorb liquid from a human body or the like. For example, an absorbent article disclosed in Patent Literature 1 includes an absorption layer including a base material and a highly absorbent resin. The base material is constituted by a fiber sheet such as a nonwoven fabric. The highly absorbent resin is held by the base material and has high liquid absorption performance. Such an absorbent article, which is attached to be in close contact with a skin surface of a human body, can absorb liquid such as urine from the human body or the like by the absorption layer.

The absorbent article having the above configuration may have a structure in which a plurality of absorption layers are superimposed in order to increase an absorption amount.

However, in the structure in which the absorption layers are superimposed, a first absorption layer close to the skin surface of the human body first absorbs water, and the highly absorbent resin swells, and thus a so-called gel block phenomenon may occur that inhibits a flow of the liquid to a second and subsequent absorption layers located downstream of the first absorption layer (non-skin surface side) in a liquid supply direction. When the gel block phenomenon occurs, supply of the liquid to the absorption layer on a downstream side is stagnant, and thus there is a problem that the absorption capacity of the absorption layer on the downstream side cannot be sufficiently utilized.

### Citation List

### Patent Literature

Patent Literature 1: International Publication WO 2017-131014 A

### Summary of Invention

The present invention has been made in view of the above circumstances, and an object of the present invention is to provide an absorbent article capable of fully utilizing absorption capacity of an absorption layer downstream in a liquid supply direction in a structure in which a plurality of absorption layers are laminated.

In order to solve the above problems, an absorbent article of the present invention absorbs liquid supplied from upstream to downstream in a predetermined supply direction, the absorbent article including a first absorption layer configured to absorb the liquid, a second absorption layer disposed downstream of the first absorption layer in the supply direction and configured to absorb the liquid, and a diffusion layer that is interposed between the first absorption layer and the second absorption layer and diffuses the liquid in a direction in which a plane of the second absorption layer spreads, in which the first absorption layer contains an absorbent resin configured to absorb the liquid, and the first absorption layer includes an absorption region containing the absorbent resin, and a passage region that has a lower density of the absorbent resin than a density of the absorbent resin in the absorption region, the passage region allowing passage of the liquid to the diffusion layer.

### Brief Description of Drawings

FIG. 1 is a plan view illustrating an overall configuration of a C-wrap absorbent article in which a passage region is formed by a space according to a first embodiment of the present invention.
FIG. 2 is a cross-sectional view of the absorbent article in FIG. 1.
FIGS. 3A to 3D are step explanatory diagrams illustrating a procedure of a method of forming a first absorber and a second absorber in FIG. 2.
FIG. 4 is a plan view illustrating an overall configuration of a G-wrap absorbent article in which a passage region is formed by a space according to a modification of the first embodiment of the present invention.
FIG. 5 is a cross-sectional view of the absorbent article in FIG. 4.
FIG. 6 is a plan view illustrating an overall configuration of a C-wrap absorbent article in which a passage region is constituted by a portion of a base material that does not hold an absorbent resin according to a second embodiment of the present invention.
FIG. 7 is a cross-sectional view of the absorbent article in FIG. 6.
FIGS. 8A to 8D are step explanatory diagrams illustrating a procedure of a method of forming the first absorber in FIG. 7.
FIG. 9 is a plan view illustrating an overall configuration of a G-wrap absorbent article in which a passage region according to a modification of the second embodiment of the present invention is constituted by a portion of a base material that does not hold an absorbent resin.
FIG. 10 is a cross-sectional view of the absorbent article in FIG. 9.

### Description of Embodiments

Hereinafter, a preferred embodiment of the present invention will be described in detail with reference to the accompanying drawings.

### (First Embodiment)

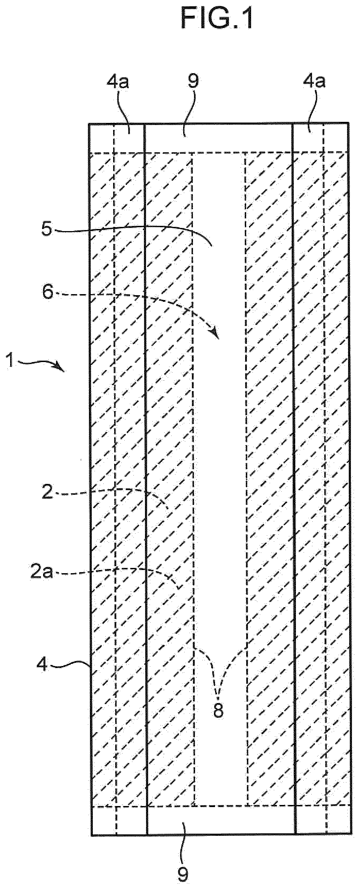

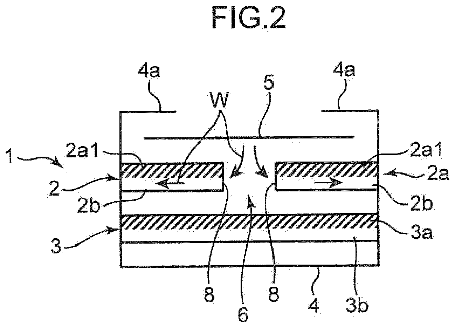

An absorbent article 1 according to a first embodiment of the present invention illustrated in FIGS. 1 and 2 has a configuration capable of absorbing liquid supplied from upstream to downstream in a predetermined supply direction (from an upper end to a lower end of the absorbent article 1 in FIG. 2). Specifically, the absorbent article 1 includes a first absorber 2, a second absorber 3, an exterior sheet 4, and a top sheet 5.

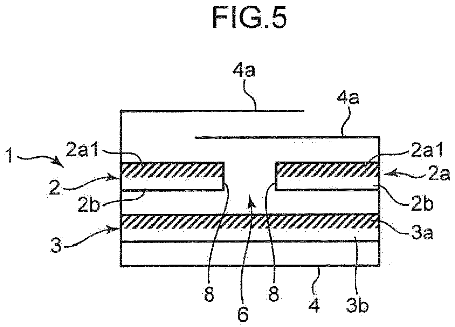

In FIG. 2, components of the absorbent article 1 are slightly separated in a vertical direction to facilitate understanding of individual forms of the components. However, in the actual absorbent article 1, the components are in close contact with each other. The same applies to FIGS. 5, 7, and 9 described later.

The absorbent article 1 illustrated in FIGS. 1 and 2 has a so-called C-wrap configuration in which the top sheet 5, the first absorber 2, and the second absorber 3 are covered in a C-shape by the exterior sheet 4 in a state where the top sheet 5, the first absorber 2, and the second absorber 3 are laminated in the vertical direction. Both ends 9 in a longitudinal direction of the absorbent article 1 illustrated in FIGS. 1 and 2 are covered with the exterior sheet 4 or another sheet. Folded portions 4a at both ends of the exterior sheet 4 in FIG. 2 are fixed to the top sheet 5 by adhesion or the like.

The exterior sheet 4 and the top sheet 5 have a property of being liquid-permeable, and are produced with a nonwoven fabric, thin paper (tissue), or the like. With the C-wrap configuration in FIGS. 1 to 2, liquid can pass through a gap between the folded portions 4a at both ends of the exterior sheet 4, and thus the exterior sheet 4 may be produced with a resin or the like that is impervious to liquid.

The absorbent article 1 has a structure in which the top sheet 5 contacts a liquid supply side, for example, a skin surface of a human body, and liquid such as urine from the human body or the like passes through the first absorber 2 and then the second absorber 3 sequentially through the top sheet 5 (that is, passes downward in FIG. 2).

The first absorber 2 includes a first absorption layer 2a configured to absorb liquid, and includes a diffusion layer 2b. The first absorption layer 2a is partially formed on a surface of the first absorber 2 facing upstream in the supply direction (upper side in FIG. 2). As a result, a portion on an upper surface of the first absorber 2 illustrated in FIG. 2 constitutes the first absorption layer 2a, and a portion on a lower surface of the first absorber 2 constitutes the diffusion layer 2b.

The first absorber 2 is divided into two by a slit 8, and a space 6 serving as a passage region for liquid is formed inside the slit 8 (that is, between the two divided portions of the first absorber 2).

Thus, the first absorption layer 2a is a layer containing an absorbent resin configured to absorb liquid, and has a structure including an absorption region 2a1 containing an absorbent resin and a space 6 as a passage region that allows passage of liquid to the diffusion layer 2b. The space 6, in which an absorbent resin is absent, forms a passage region having a lower density of absorbent resin than a density of the absorbent resin in the absorption region 2a1. The space 6 serving as the passage region penetrates the first absorber 2 in a thickness direction of the first absorber 2 (vertical direction in FIG. 2).

The space 6 according to the first embodiment is formed over an entire length of the absorbent article 1 (an entire length in the longitudinal direction of the absorbent article 1 illustrated in FIG. 1), but may be formed only in a part of the entire length of the absorbent article 1. In addition, the space 6 may have not only a band shape extending in the longitudinal direction of the absorbent article 1 (vertical direction in FIG. 1) but also a band shape extending in a width direction of the absorbent article 1 or may be a plurality of dotted through holes.

A thickness of the absorption region 2a1 containing an absorbent resin is preferably set to be smaller than a width of the space 6 (width in a lateral direction in FIG. 2). This constitution reduces a possibility that the space 6 is closed when the absorption region 2a1 absorbs water and expands.

The diffusion layer 2b is interposed between the first absorption layer 2a and a second absorption layer 3a (to be described later) of the second absorber 3, and is a layer that diffuses liquid in a direction in which a plane of the second absorption layer 3a spreads. The diffusion layer 2b spreads so as to cover an entire range of the second absorption layer 3a.

The second absorber 3 is disposed so as to be laminated downstream of the first absorber 2 in the supply direction (lower side in FIG. 2).

The second absorber 3 includes the second absorption layer 3a containing an absorbent resin configured to absorb liquid, and a base layer 3b containing no absorbent resin. The second absorption layer 3a is disposed downstream of the first absorption layer 2a in the supply direction with the diffusion layer 2b interposed between the first absorption layer 2a and the second absorption layer 3a. The second absorption layer 3a has a size that covers an entire area of the first absorption layer 2a.

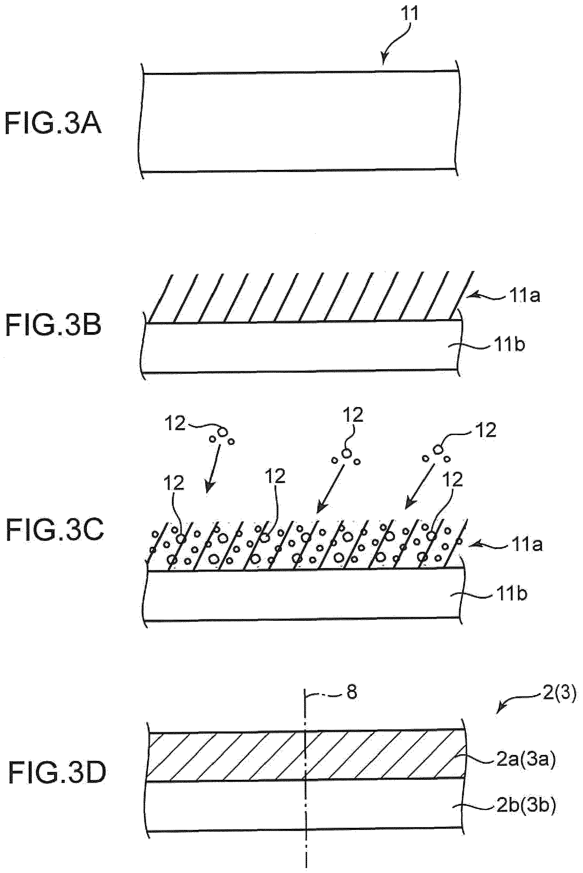

The first absorber 2 and the second absorber 3 are produced by a procedure illustrated in FIGS. 3A to 3D.

First, as shown in FIGS. 3A to 3B, an upper side of a base material 11 including a nonwoven fabric or the like having water permeability is napped to form a napped portion 11a in which short fibers constituting the base material 11 are raised. At this time, a lower portion 11b of the base material 11 supports the short fibers of the napped portion 11a.

Next, as shown in FIG. 3C, the particulate absorbent resin 12 is dispersed and held (carried or captured) all over napped portion 11a. At this time, an adhesive may be sprayed such that the absorbent resin 12 does not fall off from the napped portion 11a.

As shown in FIG. 3D, the absorbent resin 12 is held to form the first absorber 2 including the first absorption layer 2a and the diffusion layer 2b, that is, the first absorption layer 2a constituted by the napped portion 11a and containing an absorbent resin, and the diffusion layer 2b configured by the lower portion 11b and including only the nonwoven fabric containing no absorbent resin. Thereafter, the first absorber 2 is divided into two by the slit 8 to form the space 6 serving as a passage region.

Although the diffusion layer 2b, which is formed only by a nonwoven fabric, only needs to have a lower density of absorbent resin than a density of the absorbent resin in the first absorption layer 2a.

Further, it is possible to form the second absorber 3 including the second absorption layer 3a containing an absorbent resin and the base layer 3b including only a nonwoven fabric containing no absorbent resin but by a similar procedure to the procedure of the first absorber 2. In the second absorber 3, the slit 8 is not formed.

### (Characteristics of First Embodiment)

(1)
The absorbent article 1 according to the first embodiment includes the first absorption layer 2a configured to absorb liquid, the second absorption layer 3a disposed downstream of the first absorption layer 2a in the supply direction (lower side in FIG. 2) and configured to absorb liquid, and the diffusion layer 2b that is interposed between the first absorption layer 2a and the second absorption layer 3a and diffuses liquid in the direction in which the plane of the second absorption layer 3a spreads. The first absorption layer 2a contains an absorbent resin configured to absorb liquid. The first absorption layer 2a includes the absorption region 2a1 containing an absorbent resin, and the space 6 as a passage region having a lower density of absorbent resin than a density of the absorbent resin in the absorption region 2a1 and allowing passage of liquid to the diffusion layer 2b.

In this configuration, when liquid is supplied to the first absorption layer 2a from upstream to downstream in a predetermined liquid supply direction (from the upper end to the lower end of the absorbent article 1 in FIG. 2), part of the liquid is absorbed in the absorption region 2a1 of the first absorption layer 2a, and the remaining liquid passes through the space 6 serving as a passage region of the first absorption layer 2a and is supplied to the diffusion layer 2b interposed between the first absorption layer 2a and the second absorption layer 3a as indicated by an arrow W in FIG. 2. Specifically, when liquid is supplied to the first absorption layer 2a, the liquid passes through not only the space 6 but also the first absorption layer 2a and is supplied to the diffusion layer 2b (and the second absorption layer 3a downstream of the diffusion layer 2b) until the absorbent resin of the first absorption layer 2a swells to fill the gap in the first absorption layer 2a.

The liquid supplied to the diffusion layer 2b is diffused by the diffusion layer 2b in the direction in which the plane of the second absorption layer 3a spreads, and is absorbed by the second absorption layer 3a. As a result, it is possible to sufficiently utilize absorption capacity of the second absorption layer 3a downstream in the liquid supply direction without being affected by a gel block that obstructs a flow of the liquid in the first absorption layer 2a on an upstream side.

(2)
In the absorbent article 1 according to the first embodiment, the first absorber 2 includes the first absorption layer 2a. The second absorber 3 is disposed so as to be laminated downstream of the first absorber 2 in the supply direction, and includes the second absorption layer 3a. The first absorption layer 2a is included in a portion of the first absorber 2 facing upstream in the supply direction. The diffusion layer 2b is included in a lower portion of the first absorber 2 as a part of at least one of the first absorber 2 and the second absorber 3 in a part where the first absorber 2 and the second absorber 3 face each other.

In this configuration, the first absorption layer 2a is included in a portion of the first absorber 2 facing upstream in the supply direction, and the diffusion layer 2b is included in a part of at least one of the first absorber 2 and the second absorber 3 in a part where the first absorber 2 and the second absorber 3 face each other. As a result, by layering the first absorber 2 and the second absorber 3, it is possible to easily produce the absorbent article 1 in which the first absorption layer 2a, the diffusion layer 2b, and the second absorption layer 3a are laminated.

Note that the diffusion layer 2b may be included in a part of at least one of the first absorber 2 and the second absorber 3 in a part where the first absorber 2 and the second absorber 3 face each other. Therefore, instead of including the diffusion layer 2b in the lower portion of the first absorber 2 as illustrated in FIG. 2, the diffusion layer may be included in an upper portion of the second absorber 3 and the second absorption layer 3a may be included in a lower portion of the second absorber 3. Alternatively, the diffusion layer may be included in both the lower portion of the first absorber 2 and the upper portion of the second absorber 3.

(3)
In the absorbent article 1 according to the first embodiment, the first absorber 2 includes the base material 11 and the absorbent resin 12, and the first absorption layer 2a includes the absorbent resin 12 and the napped portion 11a as a holding portion that holds the absorbent resin 12 in the base material 11. The passage region is formed by the space 6 penetrating the first absorber 2 in the thickness direction of the first absorber.

In this configuration, the passage region can be easily formed by the space 6 penetrating the first absorber 2 in the thickness direction without adjusting the density of the absorbent resin 12. Furthermore, since the passage region is formed by the space 6, the arrangement and size of the passage region can be easily changed, and a degree of freedom in design is high.

(4)
In the absorbent article 1 according to the first embodiment, the space 6 is formed by the slit 8 dividing the first absorber 2 into a plurality of parts.

In this configuration, the space 6 serving as the passage region is formed by the slit 8 dividing the first absorber 2 into the plurality of parts. Thus, the space 6 having a groove shape and serving as the passage region can be easily formed by the slit 8. Moreover, the width of the space 6 can be easily changed, and the degree of freedom in design is still higher.

(5)
In the absorbent article 1 according to the first embodiment, as illustrated in FIGS. 3A to 3D, the base material 11 of the first absorber 2 has the napped portion 11a in which the base material 11 is napped as a holding portion that holds the absorbent resin 12. The absorbent resin 12 is held by the napped portion 11a.

In this configuration, the absorbent resin 12, which is held by the napped portion 11a in the first absorber 2, is less likely to fall off from the base material 11. Thus, the absorbent resin 12 can be stably held by the base material 11. In addition, a possibility that the absorbent resin 12 moves to the space 6 as the passage region is also reduced.

(6)
In the absorbent article 1 according to the first embodiment, as illustrated in FIG. 2, the diffusion layer 2b spreads so as to cover the entire range of the second absorption layer 3a. Therefore, liquid can be smoothly diffused into the entire range of the second absorption layer 3a via the diffusion layer 2b, and the absorption capacity of the second absorption layer 3a can be sufficiently utilized.

(7) In the absorbent article 1 according to the first embodiment, the space 6 as the passage region and the second absorption layer 3a are disposed so as to be laminated in the supply direction. Therefore, the liquid can smoothly flow from the passage region (space 6) to the second absorption layer 3a.

### (Modification of First Embodiment)

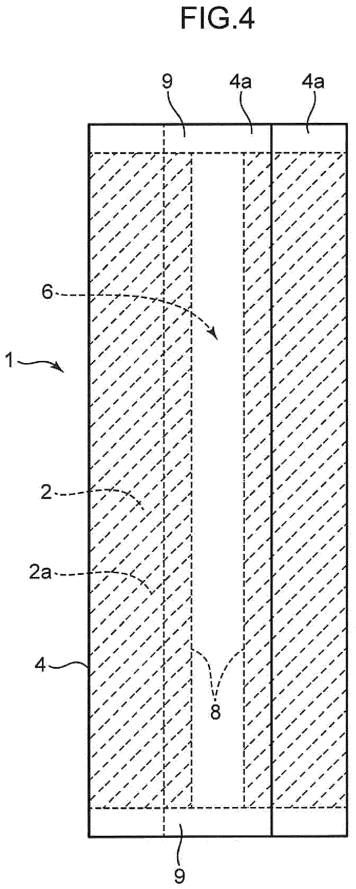

(A) In the first embodiment, the absorbent article 1 includes the first absorber 2 and the second absorber 3, the first absorber 2 includes the first absorption layer 2a and the diffusion layer 2b, and the second absorber 3 includes the second absorption layer 3a. However, the present invention is not limited to this configuration. As a modification of the present invention, for example, the first absorption layer, the diffusion layer, and the second absorption layer may be integrally formed as one member, or may be formed and laminated as separate members.
(B) The diffusion layer 2b according to the first embodiment is constituted by a layer containing no absorbent resin in the base material 11 including a nonwoven fabric shown in FIGS. 3A and 3B, but the present invention is not limited to this configuration, and other configurations may be adopted as long as the layer can diffuse liquid in the direction in which the plane of the second absorption layer 3a spreads. Therefore, as a modification of the present invention, the diffusion layer may be formed by a layer including a pipe-shaped passage or a hole.
(C) The absorbent article 1 according to the first embodiment has a C-wrap configuration in which the top sheet 5, the first absorber 2, and the second absorber 3 are laminated and covered with the exterior sheet 4 in a C-shape. However, the present invention is not limited to this configuration, and may have a G-wrap configuration without use of the top sheet 5 as illustrated in FIGS. 4 and 5. That is, as shown in FIGS. 4 and 5, the folded portions 4a on both sides of the exterior sheet 4 may overlap each other to form a G-wrap configuration covering the upper surface of the first absorber 2. This configuration can omit the top sheet 5 and simplify the structure of the absorbent article 1.

### (Second Embodiment)

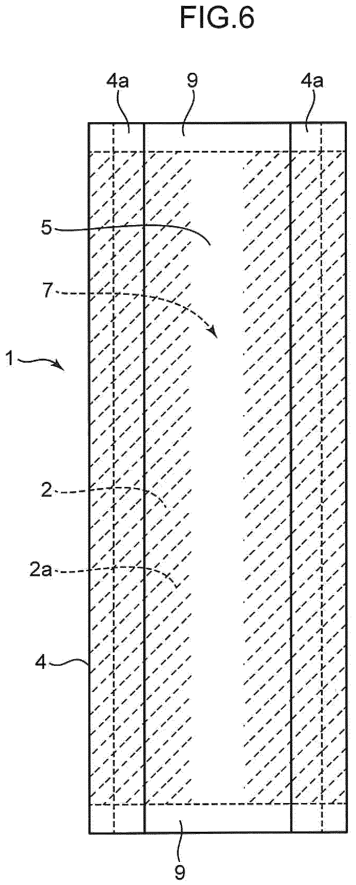

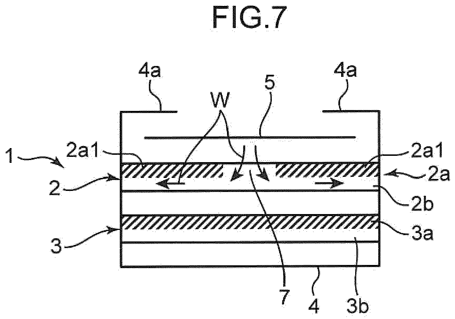

An absorbent article 1 according to a second embodiment of the present invention illustrated in FIGS. 6 and 7 is different from the absorbent article 1 according to the first embodiment in that a passage region is constituted by a passage region 7 constituted by the base material of the first absorber 2 instead of the space 6. In other respects, the configuration of the absorbent article 1 according to the second embodiment, which is common to the configuration of the absorbent article 1 according to the first embodiment, will not be described.

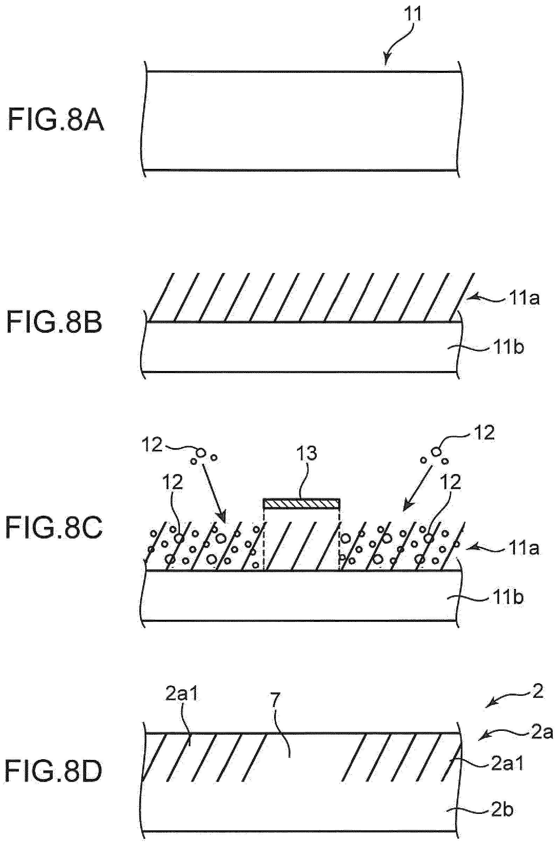

That is, in the second embodiment, the first absorption layer 2a includes the absorbent resin 12 and the napped portion 11a as a holding portion that holds the absorbent resin 12 in the base material 11. The first absorber 2 has the passage region 7 in which the density of the absorbent resin is adjusted in the first absorption layer 2a to be lower than the density of the absorbent resin in the absorption region 2a1. For example, as described later, by shielding only a portion to be the passage region 7 with a mask 13 such that the absorbent resin is not dispersed (see FIG. 8C), the passage region 7 contains no absorbent resin, and thus the density of the absorbent resin can be made lower than the density of the absorbent resin in the absorption region 2a1. Further, by using a mesh or the like instead of the mask 13, it is also possible to disperse the absorbent resin in the passage region 7 at a density lower than the density in the absorption region 2a1.

The passage region 7 according to the second embodiment is formed over an entire length of the absorbent article 1 (an entire length in the longitudinal direction of the absorbent article 1 illustrated in FIG. 6), but may be formed only in a part of the entire length of the absorbent article 1. In addition, the passage region 7 may have not only a band shape extending in the longitudinal direction of the absorbent article 1 but also a band shape extending in the width direction of the absorbent article 1 or may be a plurality of dotted regions.

The passage region 7 illustrated in FIG. 6 is formed as a band-shaped region extending in the direction of the absorbent article 1 in a plane of the first absorption layer 2a.

The first absorber 2 according to the second embodiment is produced by a procedure illustrated in FIGS. 8A to 8D.

First, as shown in FIGS. 8A to 8B, similarly to FIGS. 3A to 3B of the first embodiment, the upper side of the base material 11 including a nonwoven fabric or the like having water permeability is napped to form a napped portion 11a in which short fibers constituting the base material 11 are raised. At this time, the lower portion 11b of the base material 11 supports the short fibers.

Next, as shown in FIG. 8C, the mask 13 is placed above a predetermined portion to be the passage region 7, and in this state, the particulate absorbent resin 12 is partially dispersed and held only in a portion to be the absorption region 2a1 in the napped portion 11a.

As shown in FIG. 8D, this forms the first absorber 2 including the first absorption layer 2a and the diffusion layer 2b. The first absorption layer 2a includes the absorption region 2a1 including the napped portion 11a and the absorbent resin 12 and includes the passage region 7 containing no absorbent resin. The diffusion layer 2b is constituted by the lower portion 11b and includes only a nonwoven fabric containing no absorbent resin.

This makes it possible to produce the first absorber 2 according to the second embodiment, the first absorber 2 including the passage region 7 constituted by the base material 11 including nonwoven fabric.

### (Characteristics of Second Embodiment)

(1)
In the absorbent article 1 according to the second embodiment, the first absorber 2 has the passage region 7 in which the density of the absorbent resin is adjusted in the first absorption layer 2a to be lower than the density of the absorbent resin in the absorption region 2a1. Therefore, when liquid is supplied to the first absorption layer 2a from upstream to downstream in a predetermined liquid supply direction (from the upper end to the lower end of the absorbent article 1 in FIG. 7), part of the liquid is absorbed in the absorption region 2a1 of the first absorption layer 2a, and the remaining liquid passes through the passage region 7 of the first absorption layer 2a and is supplied to the diffusion layer 2b interposed between the first absorption layer 2a and the second absorption layer 3a as indicated by an arrow W in FIG. 7.

Specifically, also in the second embodiment, when liquid is supplied to the first absorption layer 2a, the liquid passes through not only the passage region 7 but also the absorption region 2a1 of the first absorption layer 2a and is supplied to the diffusion layer 2b (and the second absorption layer 3a downstream of the diffusion layer 2b) until the absorbent resin of the first absorption layer 2a swells to fill the gap in the first absorption layer 2a.

The liquid supplied to the diffusion layer 2b is diffused by the diffusion layer 2b in the direction in which the plane of the second absorption layer 3a spreads, and is absorbed by the second absorption layer 3a. As a result, it is possible to sufficiently utilize absorption capacity of the second absorption layer 3a downstream in the liquid supply direction without being affected by a gel block that obstructs a flow of the liquid in the first absorption layer 2a on an upstream side.

(2)
In the absorbent article 1 according to the second embodiment, the first absorber 2 includes the base material 11 and the absorbent resin 12. The first absorption layer 2a includes the absorbent resin 12 and the napped portion 11a as a holding portion that holds the absorbent resin 12 in the base material 11. The passage region 7 is a part in which the density of the absorbent resin 12 is adjusted in the first absorption layer 2a to be lower than the density of the absorbent resin 12 in the absorption region 2a1. Therefore, by partially adjusting the density of the absorbent resin 12 to be held by the base material 11 of the first absorber 2, the first absorption layer 2a including the absorption region 2a1 and the passage region 7 can be easily formed.

(3)
In the absorbent article 1 according to the second embodiment, the passage region 7 is a band-shaped region extending in the plane of the first absorption layer 2a. Thus, the passage region 7 can be formed easily in a wide range in the first absorption layer 2a. Further, by changing a width of the band-shaped passage region 7, an amount of liquid passing through the passage region 7 can be easily changed.

(4)
In the absorbent article 1 according to the second embodiment, a common configuration to the configuration of the absorbent article 1 according to the first embodiment can achieve functions and effects (characteristics of the first embodiment) of (2) and (5) to (7) described above.

### (Modification of Second Embodiment)

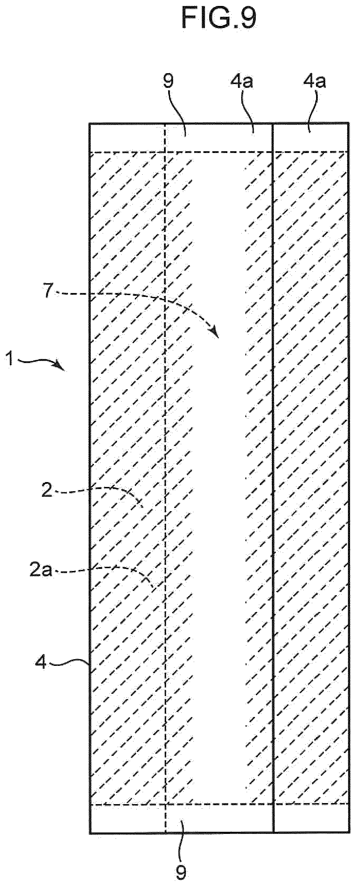

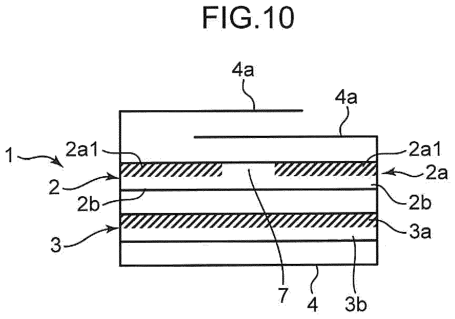

(A) In the second embodiment, similarly to the first embodiment, as a modification of the present invention, the first absorption layer, the diffusion layer, and the second absorption layer may be integrally formed as one member, or may be formed and laminated as separate members.
(B) In the second embodiment, similarly to the first embodiment, as a modification of the present invention, the diffusion layer may be formed by a layer including a pipe-shaped passage or a hole.
(C) In the second embodiment, similarly to the first embodiment, a G-wrap configuration without use of the top sheet 5 may be adopted as illustrated in FIGS. 9 and 10. That is, as shown in FIGS. 9 and 10, the folded portions 4a on both sides of the exterior sheet 4 may overlap each other to form a G-wrap configuration covering the upper surface of the first absorber 2. This configuration can omit the top sheet 5 shown in FIGS. 6 and 7 and simplify the structure of the absorbent article 1.

### <Summary of Embodiments>

The above embodiments are summarized as follows.

An absorbent article according to the embodiment absorbs liquid supplied from upstream to downstream in a predetermined supply direction, the absorbent article including a first absorption layer configured to absorb the liquid, a second absorption layer disposed downstream of the first absorption layer in the supply direction and configured to absorb the liquid, and a diffusion layer that is interposed between the first absorption layer and the second absorption layer and diffuses the liquid in a direction in which a plane of the second absorption layer spreads, in which the first absorption layer contains an absorbent resin configured to absorb the liquid, and the first absorption layer includes an absorption region containing the absorbent resin, and a passage region that has a lower density of the absorbent resin than a density of the absorbent resin in the absorption region, the passage region allowing passage of the liquid to the diffusion layer.

In such a configuration, when the liquid is supplied to the first absorption layer from upstream to downstream in a predetermined supply direction, part of the liquid is absorbed in the absorption region of the first absorption layer, and the remaining liquid passes through the passage region of the first absorption layer and is supplied to the diffusion layer interposed between the first absorption layer and the second absorption layer. The liquid supplied to the diffusion layer is diffused by the diffusion layer in the direction in which the plane of the second absorption layer spreads, and is absorbed by the second absorption layer. As a result, it is possible to sufficiently utilize absorption capacity of the second absorption layer downstream in the liquid supply direction without being affected by a gel block that obstructs a flow of the liquid in the first absorption layer on the upstream side.

The absorbent article preferably further includes a first absorber including the first absorption layer, and a second absorber disposed to be laminated downstream of the first absorber in the supply direction and including the second absorption layer, in which the first absorption layer is preferably included in a part of the first absorber facing upstream in the supply direction, and the diffusion layer is preferably included in a part of at least one of the first absorber and the second absorber in a part where the first absorber and the second absorber face each other.

In this configuration, the first absorption layer is included in a portion of the first absorber facing upstream in the supply direction, and the diffusion layer is included in a part of at least one of the first absorber and the second absorber in a part where the first absorber and the second absorber face each other. As a result, by layering the first absorber and the second absorber, it is possible to easily produce the absorbent article in which the first absorption layer, the diffusion layer, and the second absorption layer are laminated.

In the absorbent article, the first absorber preferably includes a base material and the absorbent resin, the first absorption layer preferably includes the absorbent resin and a holding portion that holds the absorbent resin in the base material, and the passage region is preferably formed by a space penetrating the first absorber in a thickness direction of the first absorber.

In this configuration, the passage region can be easily formed by the space penetrating the first absorber in the thickness direction without adjusting the density of the absorbent resin. Furthermore, since the passage region is formed by the space, the arrangement and size of the passage region can be easily changed, and a degree of freedom in design is high.

In the absorbent article described above, the space is preferably formed by a slit that divides the first absorber into a plurality of parts.

In this configuration, the space serving as the passage region is formed by the slit dividing the first absorber into the plurality of parts. Thus, the space having a groove shape and serving as the passage region can be easily formed by the slit. Moreover, the width of the space can be easily changed, and the degree of freedom in design is still higher.

In the absorbent article, the first absorber preferably includes a base material and the absorbent resin, the first absorption layer preferably includes the absorbent resin and a holding portion that holds the absorbent resin in the base material, and the passage region is preferably a portion of the first absorption layer, a density of the absorbent resin in the portion being adjusted to be lower than a density of the absorbent resin in the absorption region.

In this configuration, the first absorption layer includes the absorbent resin and the holding portion that holds the absorbent resin in the base material. Moreover, the passage region is a part in which the density of the absorbent resin is adjusted in the first absorption layer to be lower than the density of the absorbent resin in the absorption region. Therefore, by partially adjusting the density of the absorbent resin to be held by the base material of the first absorber, the first absorption layer including the absorption region and the passage region can be easily formed.

In the absorbent article, the passage region is preferably a band-shaped region extending in a plane of the first absorption layer.

In this configuration, the passage region can be formed easily in a wide range in the first absorption layer. Further, by changing a width of the band-shaped passage region, an amount of liquid passing through the passage region can be easily changed.

In the absorbent article, the holding portion is preferably a napped portion in which the base material is napped, and the absorbent resin is preferably held by the napped portion.

In this configuration, the absorbent resin, which is held by the napped portion in the first absorber, is less likely to fall off from the base material. Thus, the absorbent resin can be stably held by the base material. In addition, a possibility that the absorbent resin moves to the passage region is also reduced.

In the absorbent article, the diffusion layer preferably extends to cover an entire range of the second absorption layer.

In this configuration, since the diffusion layer spreads to cover the entire range of the second absorption layer, the liquid can be smoothly diffused into the entire range of the second absorption layer via the diffusion layer, and the absorption capacity of the second absorption layer can be sufficiently utilized.

In the absorbent article, the passage region and the second absorption layer are preferably disposed to be laminated in the supply direction.

In this configuration, the liquid can smoothly flow from the passage region to the second absorption layer.

As described above, the absorbent article according to the embodiments can fully utilize absorption capacity of an absorption layer downstream in the liquid supply direction in a structure in which a plurality of absorption layers are laminated.

## Claims

1. An absorbent article that absorbs liquid supplied from upstream to downstream in a predetermined supply direction, the absorbent article comprising:
a first absorption layer configured to absorb the liquid;
a second absorption layer disposed downstream of the first absorption layer in the supply direction and configured to absorb the liquid; and
a diffusion layer that is interposed between the first absorption layer and the second absorption layer and diffuses the liquid in a direction in which a plane of the second absorption layer spreads, wherein
the first absorption layer contains an absorbent resin configured to absorb the liquid, and
the first absorption layer includes an absorption region containing the absorbent resin and a passage region that has a lower density of the absorbent resin than a density of the absorbent resin in the absorption region, the passage region allowing passage of the liquid to the diffusion layer.

2. The absorbent article according to claim 1, further comprising:
a first absorber including the first absorption layer; and
a second absorber disposed to be laminated downstream of the first absorber in the supply direction and including the second absorption layer,
wherein the first absorption layer is included in a part of the first absorber facing upstream in the supply direction, and
the diffusion layer is included in a part of at least one of the first absorber and the second absorber in a part where the first absorber and the second absorber face each other.

3. The absorbent article according to claim 2, wherein
the first absorber includes a base material and the absorbent resin,
the first absorption layer includes the absorbent resin and a holding portion that holds the absorbent resin in the base material, and
the passage region is formed by a space penetrating the first absorber in a thickness direction of the first absorber.

4. The absorbent article according to claim 3, wherein the space is formed by a slit that divides the first absorber into a plurality of parts.

5. The absorbent article according to claim 2, wherein
the first absorber includes a base material and the absorbent resin,
the first absorption layer includes the absorbent resin and a holding portion that holds the absorbent resin in the base material, and
the passage region is a portion of the first absorption layer, a density of the absorbent resin in the portion being adjusted to be lower than a density of the absorbent resin in the absorption region.

6. The absorbent article according to claim 5, wherein the passage region is a band-shaped region extending in a plane of the first absorption layer.

7. The absorbent article according to any one of claims 3 to 6, wherein
the holding portion is a napped portion in which the base material is napped, and
the absorbent resin is held by the napped portion.

8. The absorbent article according to any one of claims 1 to 7, wherein the diffusion layer extends to cover an entire range of the second absorption layer.

9. The absorbent article according to any one of claims 1 to 8, wherein the passage region and the second absorption layer are disposed to be laminated in the supply direction.
